# DEMANDE DE BREVET EUROPEEN

(11) **EP 0 687 734 A1**
(43) Date de publication de la demande: **20.12.1995**
(21) Numéro de dépôt: 95201521.2
(22) Date de dépôt: 09.06.1995
(51) Int. Cl.: C12N 15/80, C12N 9/62, C12N 1/15

(54) **Système d'expression pour aspergillus foetidus**

(30) Priorité: 17.06.1994 BE 9400585; 09.01.1995 BE 9500015
(71) Demandeur: SOLVAY (Société Anonyme), B-1050 Bruxelles (BE)
(72) Inventeur: Carrez, Dirk, B-1820 Strombeek-Bever (BE); Dhaese, Patrick, B-9031 Gent (BE)
(74) Mandataire: Lechien, Monique

(57) **Abrégé**

L'invention concerne un système d'expression, utilisable pour la production extracellulaire de protéase aspartique microbienne, qui comprend la séquence d'un promoteur, la séquence codante de la protéase aspartique microbienne, et la séquence d'un terminateur.

L'invention concerne également un vecteur d'intégration contenant le système d'expression et une cellule transformée par ce vecteur.

LISTE DE SEQUENCES

(1) INFORMATIONS GENERALES :

(i) DEPOSANT :
(A) NOM : SOLVAY (Société Anonyme)
(B) RUE : rue du Prince Albert, 33
(C) VILLE : Bruxelles
(E) PAYS : Belgique
(F) CODE POSTAL : 1050
(G) TELEPHONE : (02) 509.61.11

(ii) TITRE DE L'INVENTION : Système d'expression, vecteur
d'intégration et cellule transformée par ce vecteur d'intégration

(iii) NOMBRE DE SEQUENCES : 8

(iv) FORME LISIBLE PAR ORDINATEUR :
(A) TYPE DE SUPPORT : Floppy disk
(B) ORDINATEUR : IBM PC compatible
(C) SYSTEME D'EXPLOITATION : PC-DOS:MS-DOS

(2) INFORMATION POUR LA SEQ ID NO:1:

(i) CARACTERISTIQUES DE LA SEQUENCE :
(A) LONGUEUR : 63 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:1:

TGATGATCAG GATCCGGCGG CCGCACCTCA GCAATGGTCG TCTTCAGCAA AACCGCTGCC 60

CTC 63

(2) INFORMATION POUR LA SEQ ID NO:2:

(i) CARACTERISTIQUES DE LA SEQUENCE :
(A) LONGUEUR : 57 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:2:

CTGGGATTCG CCGCTCAGGC TTAGATTATC AAGCTTCATT CTAGAGACGA CGACGAC 57

(2) INFORMATION POUR LA SEQ ID NO:3:

(i) CARACTERISTIQUES DE LA SEQUENCE :
(A) LONGUEUR : 57 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:3:

GTCGTCGTCG TCTCTAGAAT GAAGCTTGAT AATCTAAGCC TGAGCGGCGA ATCCCAG 57

(2) INFORMATION POUR LA SEQ ID NO:4:

(i) CARACTERISTIQUES DE LA SEQUENCE :
(A) LONGUEUR : 25 paires de bases
(B) TYPE : acide nucléique
(C) NOMBRE DE BRINS : simple
(D) CONFIGURATION : linéaire

(ii) TYPE DE MOLECULE :, acide nucléique (oligonucléotide
synthétique)

(xi) DESCRIPTION DE LA SEQUENCE: SEQ ID NO:4:

GATTCATGGT TGAGCAACGA AGCGA 25

(2) INFORMATION POUR LA SEQ ID NO:5:

(i) CARACTERISTIQUES DE LA SEQUENCE :
(A) LONGUEUR : 49 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:5:

GCCTGAGCTT CATCCCCAGC GCGGCCGCAT CATTACACCT CAGCAATGT 49

(2) INFORMATION POUR LA SEQ ID NO:6:

(i) CARACTERISTIQUES DE LA SEQUENCE :
(A) LONGUEUR : 50 paires de bases
(B) TYPE : acide nucléique
(C) NOMBRE DE BRINS : simple
(D) CONFIGURATION : linéaire

(ii) TYPE DE MOLECULE : acide nucléique (oligonucléotide
synthétique)

(xi) DESCRIPTION DE LA SEQUENCE: SEQ ID NO:6:

TGACTGACAC CTGGCGGTGA AAGCTTCAAT CAATCCATTT CGCTATAGTT 50

(2) INFORMATION POUR LA SEQ ID NO:7:

(i) CARACTERISTIQUES DE LA SEQUENCE :
(A) LONGUEUR : 2045 paires de bases
(B) TYPE : acide nucléique
(C) NOMBRE DE BRINS : simple
(D) CONFIGURATION : linéaire

(ii) TYPE DE MOLECULE : ADN génomique

(xi) DESCRIPTION DE LA SEQUENCE: SEQ ID NO:7:

GAATTCCCGA CATCGGTCAT TGGCCTCCTC GCTGATCTCC CTTTGGTACA GTCGGCTACC 60

AATGCTCTCG AGGATTGCCT GAACATTGAC ATTCGGCGTC CGGCCGGGAC CACCGCGGAC 120

TCGAAGCTGC CTGTGCTGGT CTGGATCTTT GGCGGAGGCT TTGAACTTGG TTCAAAGGCG 180

ATGTATGATG GTACAACGAT GGTATCATCG TCGATAGACA AGAACATGCC TATCGTGTTT 240

GTAGCAATGA ATTATCGCGT GGGAGGTTTC GGGTTCTTGC CCGGAAAGGA GATCCTGGAG 300

GACGGGTCCG CGAACCTAGG GCTCCTGGAC CAACGCCTTG CCCTGCAGTG GGTTGCCGAC 360

AACATCGAGG CCTTTGGTGG AGACCCGGAC AAGGTGACGA TTTGGGGAGA ATCAGCAGGA 420

GCCATTCGTT TGACTAGATG ACTTGTACGA CGGAAACATC ACTTACAAGG ATAAGCCCTT 480

GTTCCGGGGG GCCATCATGG ACTCCGGTAG TGTTGTTCCC GCAGACCCCG TCGATGGGGT 540

CAAGGGACAG CAAGTATATG ATGCGGTAGT GGAATCTGCA GGCTGTTCCT CTTCTAACGA 600

CACCCTAGCT TGTCTGCGTG AACTAGACTA CACCGACTTC CTCAATGCGG CAAACTCCGT 660

GCCAGGCATT TTAAGCTACC ATTCTGTGGC GTTATCATAT GTGCCTCGAC CGGACGGGAC 720

GGCGTTGTCG GCATCACCGG ACGTTTTGGG CAAAGCAGGG AAATATGCTC GGGTCCCGTT 780

CATCGTGGGC GACCAAGAGG ATGAGGGGAC CTTATTCGCC TTGTTTCAGT CCAACATTAC 840

GACGATCGAC GAGGTGGTCG ACTACCTGGC CTCATACTTC TTCTATGACG CTAGCCGAGA 900

GCAGCTTGAA GAACTAGTGG CCCTGTACCC AGACACCACC ACGTACGGGT CTCCGTTCAG 960

ACAGCGCGGC CAACAACTGG TATCCGCAAT TTAAGCGATT GGCCGCCATT CTCGGCGACT 1020

TGGTCTTCAC CATTACCGGC GGGCATTCCT CTCGTATGCA GAGGAAATCT CCCCTGATCT 1080

TCCGAACTGG TCGTACCTGG CGACCTATGA CTATGGCACC CCAGTTCTGG GGACCTTCCA 1140

CGGAAGTGAC CTGCTGCAGG TGTTCTATGG GATCAAGCCA AACTATGCAG CTAGTTCTAG 1200

CCACACGTAC TATCTGAGCT TTGTGTATAC GCTGGATCCG AACTCCAACC GGGGGGAGTA 1260

CATTGAGTGG CCGCAGTGGA AGGAATCGCG GCAGTTGATG AATTTCGGAG CGAACGACGC 1320

CAGTCTCCTT ACGGATGATT TCCGCAACGG GACATATGAG TTCATCCTGC AGAATACCGC 1380

GGCGTTCCAC ATCTGATGCC ATTGGCGGAG GGGTCCGGAC GGTCAGGAAC TTAGCCTTAT 1440

GAGATGAATG ATGGACGTGT CTGGCCTCGG AAAAGGATAT ATGGGATCAT GATAGTACTA 1500

GCCATATTAA TGAAGGGCAT ATACCACGCG TTGGACCTGC GTTATAGCTT CCCGTTAGTT 1560

ATAGTACCAT CGTTATACCA GCCAATCAAG TCACCACGCA CGACCGGGGA CGGCGAATCC 1620

CCGGGAATTG AAAGAAATTG CATCCCAGGC CAGTGAGGCA GCGATTGGCC ACCTCTCCAA 1680

GGCACAGGGC CATTCTGCAG CGCTGGTGGA TTCATCGCAA TTTCCCCCGG CCCGGCCCGA 1740

CACCGCTATA GGCTGGTTCT CCCACACCAT CGGAGATTCG TCGCCTAATG TCTCGTCCGT 1800

TCACAAGCTG AAGAGCTTGA AGTGGCGAGA TGTCTCTGCA GGAATTCAAG CTAGATGCTA 1860

AGCGATATTG CATGGCAATA TGTGTTGATG CATGTGCTTC TTCCTTCAGC TTCCCCTCGT 1920

GCAGATGAGG TTTGGCTATA AATTGAAGTG GTTGGTCGGG GTTCCGTGAG GGGCTGAAGT 1980

GCTTCCTCCC TTTTAGACGC AACTGAGAGC CTGAGCTTCA TCCCCAGCAT CATTACACCT 2040

CAGCA 2045

(2) INFORMATION POUR LA SEQ ID NO:8:

(i) CARACTERISTIQUES DE LA SEQUENCE :
(A) LONGUEUR : 1035 paires de bases
(B) TYPE : acide nucléique
(C) NOMBRE DE BRINS : simple
(D) CONFIGURATION : linéaire

(ii) TYPE DE MOLECULE : ADN génomique

(xi) DESCRIPTION DE LA SEQUENCE: SEQ ID NO:8:

CAATCAATCC ATTTCGCTAT AGTTAAAGGA TGGGGATGAG GGCAATTGGT TATATGATCA 60

TGTATGTAGT GGGTGTGCAT AATAGTAGTG AAATGGAAGC CAGTCATGTG ATTGTAATCG 120

ACCGACGGAA TTGAGGATAT CCGGAAATAC AGACACCGTG AAAGCCATGG TCTTTCCTTC 180

GTGTAGAAGA CCAGACAGAC AGTCCCTGAT TTACCCTTGC ACAAAGCACT AGAAAATTAG 240

CATTCCATCC TTCTCTGCTT GCTCTGCTGA TATCACTGTC ATTCAATGCA TAGCCATGAG 300

CTCATCTTAG ATCCAAGCAC GTAATTCCAT AGCCGAGGTC CACAGGTGAG CAGCAACATT 360

CCCCATCATT GCTTTCCCAG GGCCTCCCAA CGACTAAATC AAGAGTATAT CTCTACCGTC 420

CAATAGATCG TCTTCGCTTC AAAATCTTTG ACAATTCCAA GAGGTCCCCC ATCCATCAAA 480

CCCAGTTCAA TAATAGCCGA GATGCATGGT GGAGTCAATT AGGCAGTATT GCTGGAATGT 540

CGGGGCCAGT TCCGGTGGTC ATTGGCCGCC TGTGATGCCA TCTGCCACTA AATCCGATCA 600

TTGATCCACC GCCCACGAGG CGCGTCTTTG CTTTTTGCGC GGCGTCCAGG TTCAACTCTC 660

TCTGCAGCTC CAGTCCAACG CTGACTGACT AGTTTACCTA CTGGTCTGAT CGGCTCCATC 720

AGAGCTATGG CGTTATCCCG TGCCGTTGCT GCGCAATCGC TATCTTGATC GCAACCTTGA 780

ACTCACTCTT GTTTTAATAG TGATCTTGGT GACGGAGTGT CGGTGAGTGA CAACCAACAT 840

CGTGCAAGGG AGATTGATAC GGAATTGTCG CTCCCATCAT GATGTTCTTG CCGGCTTTGT 900

TGGCCCTATC GTGGGATCGG ATGCCCTCGC TGTGCAGCAG CAGGTACTGC TGGATGAGGA 960

GCCATCGGTC TCTGCACGCA AACCCAACTT CCTCTTCATT CTCACGGATG ATCAGGATCT 1020

CCGGATGAAG AATTC 1035

## Description

L'invention concerne un système d'expression utilisable pour la production extracellulaire de protéases aspartiques microbiennes et, plus particulièrement, d'aspergillopepsines.

L'invention concerne également un vecteur d'intégration contenant ce système d'expression et une cellule transformée par ce vecteur d'intégration.

Les protéases aspartiques microbiennes sont classées dans le système international selon la référence E.C. 3.4.23. Elles sont également nommées protéases acides ou protéases carboxyles. Ces enzymes hydrolysent, sous des conditions acides, les liaisons peptidiques des protéines en formant des peptides. Elles ont un pH optimum inférieur à 5. Ces enzymes ont de nombreuses applications industrielles.

Les protéases aspartiques microbiennes sont naturellement produites par certaines souches de champignons filamenteux et notamment par des souches d'Aspergillus. Malheureusement, les protéases aspartiques microbiennes ne sont pas produites par ces souches de manière efficace et industriellement rentable, leur productivité étant faible. C'est pourquoi une production efficace a longtemps été recherchée.

Dans ce contexte, on a proposé un système d'expression comprenant la séquence du promoteur de l'α-amylase d'Aspergillus oryzae, la séquence de la protéase aspartique de Mucor miehei, la séquence du terminateur de la glucoamylase d'Aspergillus niger (BIO/TECHNOLOGY (1988) 6, pages 1419-1422). Ce système introduit dans Aspergillus oryzae permet d'obtenir la protéase aspartique. Cependant, la protéase aspartique obtenue est hyperglycosylée. Ceci modifie les propriétés essentielles de cette enzyme de façon préjudiciable.

Par ailleurs, on a décrit un système d'expression utilisable pour la production de chymosine bovine. Ce système comprend la séquence du promoteur de la glucoamylase d'Aspergillus niger, la séquence du peptide signal de la glucoamylase d'Aspergillus niger, la séquence codante de la chymosine bovine et la séquence du terminateur de la glucoamylase d'Aspergillus niger (demande de brevet européen 0 357 127). Une souche transformée d'Aspergillus niger, contenant ce système d'expression, permet d'obtenir la chymosine bovine. Cependant, avec ce système d'expression, le rendement final obtenu est faible, car les protéases produites par la souche transformée hydrolysent la chymosine bovine. De plus, aucune mention n'est faite à l'application possible d'un tel système d'expression pour la production d'une enzyme microbienne et en particulier d'une protéase aspartique microbienne.

A l'heure actuelle, un système d'expression permettant d'obtenir l'expression et une sécrétion particulièrement efficace d'une protéase aspartique microbienne par une souche transformée de champignon filamenteux contenant ce système d'expression et permettant également d'obtenir une protéase aspartique microbienne normalement glycosylée, n'est pas connu.

La présente invention a pour premier but de fournir un système d'expression permettant d'obtenir une protéase aspartique microbienne, et particulièrement une protéase acide fongique, et plus particulièrement une aspergillopepsine, en grande quantité dans le milieu de culture, où une souche transformée contenant ce système d'expression est cultivée.

La présente invention a également pour premier but de fournir un système d'expression permettant d'obtenir une protéase aspartique microbienne, et particulièrement une protéase acide fongique, et plus particulièrement une aspergillopepsine, normalement glycosylée, c'est-à-dire ayant des caractéristiques et des propriétés aussi proches que possible de celles de l'enzyme native (sauvage).

Par "protéase aspartique microbienne", on entend une enzyme qui catalyse la réaction d'hydrolyse de protéines en peptides et qui a un pH optimum inférieur à 5. Ces enzymes ont un site catalytique acide. Ces enzymes sont classées dans le système international selon la référence E.C. 3.4.23 et, plus particulièrement, selon toutes les références de E.C. 3.4.23.18 à E.C. 3.4.23.33. Cette définition inclut les enzymes naturelles et les enzymes modifiées, telles que les enzymes dont la séquence de nucléotides (qui code pour cette enzyme) ou la séquence d'acides aminés a été modifiée par des techniques de génie génétique ou par des techniques de mutagénèse. Habituellement, la protéase aspartique microbienne est une protéase aspartique fongique. D'excellents résultats ont été obtenus avec l'aspergillopepsine (E.C. 3.4.23.18).

La présente invention a également pour but de fournir un vecteur d'intégration et un plasmide contenant le système d'expression décrit ci-dessus.

La présente invention a également pour but de fournir une souche transformée de champignon filamenteux, de préférence d'Aspergillus, et plus particulièrement une souche transformée d'Aspergillus foetidus, qui exprime et sécrète dans son milieu de culture la protéase aspartique microbienne avec un haut niveau de productivité. Selon l'invention, la sécrétion de la protéase aspartique microbienne est substantiellement extracellulaire.

De plus, la présente invention présente de nombreux avantages lorsque la protéase aspartique microbienne provient de la souche sauvage d'Aspergillus qui a été mise en oeuvre lors de la transformation. En effet, la transformation est, dans ce cas, parfaitement homologue et ne présente donc aucun des inconvénients qu'entraine une transformation hétérologue, telle que notamment une hyperglycosylation de l'enzyme. Par "transformation homologue", on entend un mode de transfert génétique mettant en jeu une cellule microbienne et une molécule d'ADN qui pénètre cette cellule et peut recombiner le génome cellulaire, la molécule d'ADN provenant d'une cellule microbienne appartenant au même genre. De préférence, la molécule d'ADN provient d'une cellule microbienne appartenant à la même espèce. De manière particulièrement préférée, la molécule d'ADN provient de la cellule microbienne elle-même.

La présente invention a également pour but de fournir un procédé de production de protéase aspartique microbienne mettant en oeuvre une souche de champignon filamenteux qui produit une grande quantité de protéase aspartique microbienne de façon extracellulaire, de plus la protéase aspartique microbienne obtenue est glycosylée de la même manière que l'enzyme native.

La présente invention a également pour but de fournir une molécule d'ADN comprenant la séquence de nucléotides qui code pour le promoteur de la glucoamylase d'Aspergillus foetidus.

La présente invention a également pour but de fournir une molécule d'ADN comprenant la séquence de nucléotides qui code pour la protéase aspartique microbienne, et particulièrement pour la protéase acide fongique, et plus particulièrement pour l'aspergillopepsine.

La présente invention a également pour but de fournir une molécule d'ADN comprenant la séquence de nucléotides qui code pour l'aspergillopepsine dérivée d'une souche d'Aspergillus foetidus, et particulièrement de la souche d'Aspergillus foetidrns SE4.

La présente invention a également pour but de fournir une protéase aspartique microbienne, et particulièrement une protéase acide fongique et plus particulièrement une aspergillopepsine produite par une souche d'Aspergillus foetidus, et particulièrement par la souche d'Aspergillus foetidus SE4.

La présente invention a également pour but de fournir une protéase aspartique microbienne produite par une souche transformée comprenant le système d'expression. La souche peut être une souche d'Aspergillus foetidus, et particulièrement de la souche d'Aspergillus foetidus SE4.

A cet effet, l'invention concerne un système d'expression utilisable pour la production extracellulaire d'une protéase aspartique microbienne, caractérisé en ce qu'il comprend :
- la séquence d'un promoteur,
- la séquence d'un signal de sécrétion,
- la séquence d'un propeptide, et
- la séquence mature de la protéase aspartique microbienne.

De préférence, le système d'expression comprend également la séquence d'un terminateur.

De préférence, dans le système d'expression selon l'invention la séquence du promoteur est celle du promoteur de la glucoamylase.

L'invention concerne un système d'expression utilisable pour la production extracellulaire d'une protéase aspartique microbienne, caractérisé en ce qu'il comprend au moins :
- la séquence du promoteur de la glucoamylase,
- la séquence du signal de sécrétion de la protéase aspartique microbienne,
- la séquence du propeptide de la protéase aspartique microbienne,
- la séquence mature de la protéase aspartique microbienne, et
- la séquence d'un terminateur.

De préférence, dans le système d'expression selon l'invention la séquence du terminateur est celle du terminateur de la glucoamylase.

L'invention concerne, de préférence, un système d'expression utilisable pour la production extracellulaire d'une protéase acide fongique, caractérisé en ce qu'il comprend au moins :
- la séquence du promoteur de la glucoamylase d'Aspergillus foetidus SE4,
- la séquence du signal de sécrétion de la protéase acide fongique d'Aspergillus foetidus SE4,
- la séquence du propeptide de la protéase acide fongique d'Aspergillus foetidus SE4,
- la séquence mature de la protéase acide fongique d'Aspergillus foetidus SE4, et
- la séquence du terminateur de la glucoamylase d'Aspergillus foetidus SE4.

Par "système d'expression", on entend une unité moléculaire capable d'être intégrée dans le génome d'un champignon filamenteux et capable de fournir à ce champignon l'information génétique nécessaire à la biosynthèse de la protéase aspartique microbienne.

Par "promoteur", on entend le ou les promoteur(s) de transcription. Le promoteur qui est constitué d'une séquence d'ADN montrant une forte activité de transcription, peut être précédé par des séquences d'ADN qui stimulent la transcription, telles que des séquences connues sous le nom de "enhancers" ou "Upstream Activating Sequences". Le promoteur contient des séquences de contrôle de transcription qui influent sur l'expression de l'ADN codant fusionné. La séquence du promoteur d'un gène, telle que du promoteur de la glucoamylase, comprend les séquences qui contrôlent la transcription et qui interviennent lors de l'expression de ce gène, tel que la glucoamylase.

Par la "séquence du signal de sécrétion" de la protéase aspartique microbienne, on entend une séquence d'ADN correspondant à une séquence d'acides aminés qui est naturellement liée de façon opérationnelle à la terminaison aminée de la séquence du propeptide de la protéase aspartique microbienne. Dans cette demande, la partie du gène de structure, qui code pour le peptide signal, est nommée "séquence du signal de sécrétion de la protéase aspartique microbienne"; la partie du gène de structure, qui code pour le propeptide, est nommée "séquence du propeptide de la protéase aspartique microbienne"; et la partie de gène de structure, qui code pour la protéase aspartique microbienne en tant que telle, est nommée "séquence mature de la protéase aspartique microbienne". L'ensemble de ces trois séquences, c'est-à-dire séquence du signal de sécrétion, séquence du propeptide et séquence mature, code pour la préproenzyme. Dans cette demande, la séquence du signal de sécrétion peut provenir de n'importe quelle protéine qui est produite de façon extracellulaire par un microorganisme. N'importe quelle séquence de signal de sécrétion fonctionnelle dans un champignon filamenteux peut convenir. Comme exemple de séquence de signal de sécrétion d'origine fongique, on peut citer la séquence du signal de sécrétion d'une enzyme produite par un champignon filamenteux de façon substantiellement extracellulaire. Parmi celles-ci, on peut citer la séquence du signal de sécrétion d'une protéase, d'une cellulase, d'une amylase, d'une glucoamylase et, en particulier, de la glucoamylase d'Aspergillus niger, de la cellulase de Trichoderma reesei, de la protéase aspartique de Mucor miehei.

Par la "séquence du propeptide" de la protéase aspartique microbienne, on entend une séquence d'ADN correspondant à une séquence d'acides aminés qui est naturellement liée de façon opérationnelle à la terminaison aminée de la séquence mature de la protéase aspartique microbienne. La séquence du propeptide liée à la séquence mature de la protéase aspartique microbienne code pour la proenzyme ou zymogène.

Par "séquence mature" de la protéase aspartique microbienne, on entend la partie de la séquence qui est traduite en protéine active, c'est-à-dire la séquence codante de la protéase aspartique microbienne qui correspond au gène de structure de la protéase aspartique microbienne sans la séquence du signal de sécrétion et sans la séquence du propeptide. La séquence codante (gène de structure) comprend la séquence du signal de sécrétion, la séquence du propeptide et la séquence mature de l'aspergillopepsine.

Par "terminateur", on entend le ou les terminateur(s) de transcription. La séquence du terminateur d'un gène est une séquence d'ADN qui agit pour terminer la transcription de ce gène. La séquence du terminateur contient également le signal de polyadénylation qui a pour but de stabiliser le mARN. Dans cette demande, n'importe quel terminateur fonctionnel dans un champignon filamenteux peut convenir. Des terminateurs sont connus de l'homme du métier. Comme exemples de terminateur d'origine fongique connu, on peut citer le terminateur trpC, celui d'une glucoamylase, celui d'une amylase, celui d'une alpha-amylase, celui d'une protéase aspartique, celui d'une phosphatase acide, celui d'une lipase, celui d'une cellulase, celui d'une enzyme glycolytique, celui d'une glucanase, celui d'une hydrolase, celui d'une déhydrogénase, et en particulier, le terminateur trpC d'Aspergillus nidulans, le terminateur de la glucoamylase d'Aspergillus niger, celui de l'alpha-amylase d'Aspergillus niger, celui de la protéase aspartique de Mucor miehei, celui de la phosphatase acide d'Aspergillus niger, celui d'une alcooldéhydrogénase d'Aspergillus nidulans.

Habituellement, la séquence du promoteur de la glucoamylase provient d'un champignon filamenteux, de même que la séquence du signal de sécrétion de la protéase aspartique microbienne, la séquence du propeptide de la protéase aspartique microbienne, la séquence mature de la protéase aspartique microbienne et que la séquence du terminateur de la glucoamylase.

Ces séquences, c'est-à-dire la séquence du promoteur de la glucoamylase, la séquence du signal de sécrétion de la protéase aspartique microbienne, la séquence du propeptide de la protéase aspartique microbienne, la séquence mature de la protéase aspartique microbienne et la séquence du terminateur de la glucoamylase, proviennent de souches de champignons filamenteux identiques ou différentes.

Généralement, elles proviennent d'une souche d'Aspergillus, de Penicillium, de Rhizopus, d'Endothia ou de Mucor. Habituellement, elles proviennent d'une souche d'Aspergillus. De préférence, elles proviennent d'une souche d'Aspergillus niger, d'Aspergillus foetidus, d'Aspergillus awamori ou d'Aspergillus oryzae. De manière particulièrement préférée, elles proviennent d'une souche d'Aspergillus niger ou d'une souche d'Aspergillus foetidus. De préférence, elles proviennent d'une souche d'Aspergillus foetidus. De manière particulièrement préférée, elles proviennent de la souche d'Aspergillus foetidus SE4.

Des souches d'Aspergillus niger et des souches d'Aspergillus foetidus, d'où peuvent provenir l'une ou l'autre des séquences décrites précédemment, sont connues, telles que notamment la souche d'Aspergillus niger NRRL 3 (AGRICULTURAL RESEARCH SERVICE CULTURE COLLECTION (NRRL), 1815 North University Street, Peoria, Illinois 61604, USA), la souche d'Aspergillus niger ATCC 13496 (AMERICAN TYPE CULTURE COLLECTION, 12301 Parklawn drive, Rockville, Maryland 20852-1776, USA), la souche d'Aspergillus niger ATCC 22343, la souche d'Aspergillus niger ATCC 46890, la souche d'Aspergillus foetidus ATCC 10254, la souche d'Aspergillus foetidus ATCC 14916.

Dans une variante préférée de l'invention, la séquence du signal de sécrétion de la protéase aspartique microbienne, la séquence du propeptide de la protéase aspartique microbienne et la séquence mature de la protéase aspartique microbienne proviennent du même champignon filamenteux.

D'excellents résultats ont été obtenus avec un système d'expression, selon l'invention, comprenant la séquence du signal de sécrétion de l'aspergillopepsine de la souche d'Aspergillus foetidus SE4, la séquence du propeptide de l'aspergillopepsine de la souche d'Aspergillus foetidus SE4 et la séquence mature de l'aspergillopepsine de la souche d'Aspergillus foetidus SE4.

Dans une variante préférée de l'invention, la séquence du promoteur, de préférence celui de la glucoamylase, et la séquence du terminateur, de préférence celui de la glucoamylase, proviennent du même champignon filamenteux. De bons résultats ont été obtenus avec un système d'expression, selon l'invention, comprenant la séquence du promoteur de la glucoamylase de la souche d'Aspergillus foetidus et la séquence du terminateur de la glucoamylase de la souche d'Aspergillus foetidus. D'excellents résultats ont été obtenus avec un système d'expression, selon l'invention, comprenant la séquence du promoteur de la glucoamylase de la souche d'Aspergillus foetidus SE4 et la séquence du terminateur de la glucoamylase de la souche d'Aspergillus foetidus SE4. La molécule d'ADN comprenant la séquence de nucléotides SEQ ID NO:7 code pour le promoteur de la souche d'Aspergillus foetidus SE4. La molécule d'ADN comprenant la séquence de nucléotides SEQ ID NO:8 code pour le terminateur de la souche d'Aspergillus foetidus SE4.

Dans une variante particulièrement préférée de l'invention, la séquence du promoteur de la glucoamylase, la séquence du signal de sécrétion de la protéase aspartique microbienne, la séquence du propeptide de la protéase aspartique microbienne, la séquence mature de la protéase aspartique microbienne et la séquence du terminateur de la glucoamylase proviennent du même champignon filamenteux.

De préférence dans le système d'expression selon l'invention, la séquence du promoteur est positionnée en amont de la séquence du signal de sécrétion. La séquence du signal de sécrétion elle-même est positionnée en amont de la séquence du propeptide, la séquence du propeptide est positionnée en amont de la séquence mature, cette séquence mature est positionnée en amont de la séquence du terminateur. Ces positionnements sont choisis de telle sorte que, dans des conditions appropriées, ils permettent l'expression de la protéase aspartique microbienne sous le contrôle des signaux de transcription, c'est-à-dire du promoteur et du terminateur.

De préférence, les séquences comprises dans le système d'expression sont liées de façon opérationnelle. La séquence du promoteur est liée de façon opérationnelle à la séquence du signal de sécrétion de la protéase aspartique microbienne. La séquence du signal de sécrétion de la protéase aspartique microbienne est liée de façon opérationnelle à la séquence du propeptide de la protéase aspartique microbienne. La séquence du propeptide de la protéase aspartique microbienne est liée de façon opérationnelle à la séquence mature de la protéase aspartique microbienne. La séquence mature de la protéase aspartique microbienne est liée de façon opérationnelle à la séquence du terminateur.

L'invention concerne également un procédé de préparation d'un système d'expression comprenant au moins la séquence du promoteur, la séquence du signal de sécrétion de la protéase aspartique microbienne, la séquence du propeptide de la protéase aspartique microbienne, la séquence mature de la protéase aspartique microbienne, et la séquence du terminateur. Ce procédé comprend :
- l'isolement de la séquence du signal de sécrétion de la protéase aspartique microbienne, de la séquence du propeptide de la protéase aspartique microbienne et de la séquence mature de la protéase aspartique microbienne à partir de l'ADN génomique d'un microorganisme qui produit cette protéase aspartique microbienne, et
- l'introduction de la séquence du signal de sécrétion de la protéase aspartique microbienne, de la séquence du propeptide de la protéase aspartique microbienne et de la séquence mature de la protéase aspartique microbienne dans un vecteur contenant la séquence du promoteur et la séquence du terminateur, cette introduction étant faite dans un site choisi de telle sorte que le positionnement des séquences permet l'expression de la protéase aspartique microbienne sous le contrôle dudit promoteur et dudit terminateur.

L'invention concerne également un vecteur d'intégration contenant le système d'expression tel que défini ci-dessus, et apte à permettre l'expression de la protéase aspartique microbienne.

Le principe de l'invention s'applique également à un vecteur d'expression contenant le système d'expression tel que défini ci-dessus, et apte à permettre l'expression de la protéase aspartique microbienne.

Par "vecteur d'intégration", on entend toute sequence d'ADN qui comprend une unité d'expression génique complète. Par "unité d'expression génique complète", on entend le gène de structure et la ou les régions(s) du promoteur et la ou les région(s) de régulation nécessaire pour la transcription et la traduction. Par "gène de structure", on entend la séquence codante qui est utilisée pour la transcription en ARN et permet la synthèse de la protéine par l'hôte.

De préférence, ce vecteur d'intégration est un plasmide. De bons résultats ont été obtenus avec le plasmide pFASPEP.

L'invention concerne également une cellule transformée (cellule hôte) par le vecteur d'intégration défini ci-dessus. Cette cellule transformée est choisie de telle sorte que la séquence du promoteur et la séquence du terminateur comprises dans le système d'expression soient reconnues par cette cellule transformée, c'est-à-dire qu'elles sont compatibles et fonctionnelles pour cette cellule transformée, la séquence du promoteur et la séquence du terminateur comprises dans le système d'expression remplissent leurs fonctions respectives de signal de transcription, comme respectivement un promoteur et un terminateur, pour la cellule transformée.

Habituellement, la cellule transformée est une cellule de champignon filamenteux. Généralement, la cellule transformée est une cellule d'Aspergillus. De préférence, la cellule transformée est une cellule d'Aspergillus oryzae, d'Aspergillus saitoi, d'Aspergillus niger, d'Aspergillus foetidus, ou d'Aspergillus avamori. De bons résultats ont été obtenus avec une cellule transformée d'Aspergillus niger. D'excellents résultats ont été obtenus avec une cellule transformée d'Aspergillus foetidus, et plus particulièrement avec une cellule transformée de la souche d'Aspergillus foetidus SE4.

Des souches d'Aspergillus niger et des souches d'Aspergillus foetidus, qui peuvent être utilisées comme cellule à transformer (cellule hôte), sont connues, telles que notamment la souche d'Aspergillus niger NRRL 3 (AGRICULTURAL RESEARCH SERVICE CULTURE COLLECTION (NRRL), 1815 North University Street, Peoria, Illinois 61604, USA), la souche d'Aspergillus niger ATCC 13496 (AMERICAN TYPE CULTURE COLLECTION, 12301 Parklawn drive, Rockville, Maryland 20852-1776, USA), la souche d'Aspergillus niger ATCC 22343, la souche d'Aspergillus niger ATCC 46890, la souche d'Aspergillus foetidus ATCC 10254, la souche d'Aspergillus foetidus ATCC 14916.

Dans une variante préférée de l'invention, la cellule transformée par le vecteur d'intégration dérive de la souche d'où provient la séquence du promoteur ou la séquence du terminateur, séquence comprise dans le système d'expression. De manière particulièrement préférée, la cellule transformée par le vecteur d'intégration dérive de la souche d'où proviennent la séquence du signal de sécrétion de la protéase aspartique microbienne, la séquence du propeptide de la protéase aspartique microbienne et la séquence mature de la protéase aspartique microbienne. D'excellents résultats ont été obtenus avec une cellule transformée d'Aspergillus foetidus SE4. Cette cellule transformée par un vecteur d'intégration contient un système d'expression selon l'invention, ce système d'expression comprenant la séquence du promoteur de la glucoamylase d'Aspergillus foetidus SE4 et la séquence du terminateur de la glucoamylase d'Aspergillus foetidus SE4. Les meilleurs résultats ont été obtenus avec une cellule transformée par le vecteur d'intégration pFASPEP.

Par "champignon filamenteux", on entend les microorganismes eucaryotes qui comprennent toutes les formes filamenteuses de la division Eumycota. Dans cette division sont inclus les groupes Zygomycètes, Ascomycètes, Basidiomycètes et les champignons imparfaits incluant les Hyphomycètes. Les genres suivants sont compris dans cette définition : Aspergillus, Trichoderma, Neurospora, Podospora, Endothia, Mucor, Cochiobolus, Pyricularia, Penicillium, Humicola.

L'invention concerne également la souche purifiée et isolée d'Aspergillus foetidus SE4tra. Cette souche transformée SE4tra est obtenue à partir de la souche SE4. Elle diffère de cette souche SE4 par le fait qu'elle contient une ou plusieurs copies du plasmide pFASPEP intégrées dans son génome.

La souche d'Aspergillus foetidus SE4 a été obtenue à partir de la souche d'Aspergillus foetidus ATCC 14916 par mutagénèse et sélectionnée sur la base d'une production améliorée en glucoamylase.

La dénomination d'Aspergillus foetidus est synonyme d'Aspergillus citricus (ATCC Names of Industrial Fungi, 1994, p. 120, publié par The American Type Culture Collection).

L'invention concerne également le promoteur de la glucoamylase d'Aspergillus foetidus.

L'invention concerne également une molécule d'ADN comprenant la séquence de nucléotides SEQ ID NO:7 qui code pour le promoteur de la glucoamylase d'Aspergillus foetidus SE4.

L'invention concerne également le terminateur de la glucoamylase d'Aspergillus foetidus.

L'invention concerne également une molécule d'ADN comprenant la séquence de nucléotides SEQ ID NO:8 qui code pour le terminateur de la glucoamylase d'Aspergillus foetidus SE4.

L'invention concerne également la protéase aspartique microbienne dérivée d'une souche d'Aspergillus foetidus. De préférence, la protéase aspartique microbienne est une aspergillopepsine. De manière particulièrement préférée, elle dérive de la souche d'Aspergillus foetidus SE4.

L'invention concerne également une molécule d'ADN comprenant la séquence de nucléotides qui code pour une protéase aspartique microbienne dérivée d'une souche d'Aspergillus foetidus. De préférence, la protéase aspartique microbienne est une aspergillopepsine. De manière particulièrement préférée, elle dérive de la souche d'Aspergillus foetidus SE4.

L'invention concerne également une molécule d'ADN comprenant la séquence de nucléotides qui code pour l'aspergillopepsine dérivée d'une souche d'Aspergillus foetidus, et particulièrement de la souche d'Aspergillus foetidus SE4.

L'invention concerne également une protéase aspartique microbienne produite par une cellule transformée avec le système d'expression de la présente invention.

De préférence, la protéase aspartique microbienne est une protéase acide fongique. De manière particulièrement préférée, il s'agit d'une aspergillopepsine.

De préférence, la protéase aspartique microbienne dérive d'une souche d'Aspergillus foetidus. De manière particulièrement préférée, elle dérive de la souche d'Aspergillus foetidus SE4.

L'invention concerne également un procédé pour la production extracellulaire de protéase aspartique microbienne caractérisé en ce qu'il comprend les étapes suivantes :
- transformation d'une cellule par un vecteur d'intégration contenant un système d'expression tel que défini ci-dessus dans des conditions permettant l'expression et la sécrétion de la protéase aspartique microbienne,
- culture de la cellule transformée dans un milieu de culture adéquat, et
- récupération de la protéase aspartique microbienne sécrétée.

Le milieu de fermentation obtenu après la culture de la cellule transformée contient la majeure partie de la protéase aspartique microbienne. En effet, la protéase aspartique microbienne est substantiellement sécrétée dans le milieu de culture par la souche transformée. La protéase aspartique microbienne obtenue par le procédé de l'invention est extracellulaire.

La protéase aspartique microbienne obtenue par le procédé de l'invention est glycosylée de la même manière que l'enzyme native. Son poids moléculaire est proche de celui de la protéase aspartique microbienne produite par le microorganisme non transformé.

Les protéases aspartiques microbiennes ont de nombreuses applications industrielles, telles que, par exemple, dans les industries alimentaires, les industries pharmaceutiques et les industries chimiques. On exploite leur capacité à hydrolyser les protéines sous des conditions acides.

Les protéases aspartiques microbiennes peuvent être utilisées, en industries alimentaires, lors de la fabrication de certains fromages et de certains produits de panification.

Les protéases aspartiques microbiennes peuvent être utilisées comme médicament pour aider à la digestion.

Elles sont notamment employées pour épiler et dégraisser la peau de certains animaux pour obtenir des cuirs de qualité supérieure.

Les protéases aspartiques microbiennes peuvent être utilisées lors de la fabrication d'hydrolysats de protéines. Dans ce cadre, on peut citer la préparation de milieu de culture, la préparation d'hydrolysats de farine de poisson et de protéines de soja solubles.

De plus, en mélange avec la glucoamylase, les protéases aspartiques microbiennes, telles que notamment les protéases acides fongiques, sont utilisées lors de la fabrication d'éthanol à partir de matières premières brutes contenant des sucres fermentescibles (grains, résidus agricoles), telle que décrite dans la demande de brevet WO 92/20777 de SOLVAY ENZYMES, Inc.

La figure 1 représente la carte de restriction du plasmide pUCASPEP.

La figure 2 représente la carte de restriction du plasmide pFGA1.

La figure 3 représente la carte de restriction du plasmide pFGA2.

La figure 4 représente la carte de restriction du plasmide pFGA2MUT.

La figure 5 représente la carte de restriction du plasmide pFASPEP.

La figure 6 (figure 6a et figure 6b) représente la séquence de nucléotides (SEQ ID NO:7) codant pour le promoteur de la glucoamylase d'Aspergillus foetidus SE4.

La figure 7 représente la séquence de nucléotides (SEQ ID NO:8) codant pour le terminateur de la glucoamylase d'Aspergillus foetidus SE4.

Les symboles et abréviations utilisés dans ces figures sont explicités dans le tableau 1.

**TABLEAU 1**

| Symbole Abréviation | Signification |
|---|---|
| AMPR | gène apportant la résistance à l'ampicilline |
| ORI | origine de réplication dans E. coli |
| ASPEPSIN | séquence codante de l'aspergillopepsine d'Aspergillus foetidus SE4 |
| 5' GA | promoteur de la glucoamylase d'Aspergillus foetidus SE4 |
| 3' GA | terminateur de la glucoamylase d'Aspergillus foetidus SE4 |
| GA | séquence codante de la glucoamylase d'Aspergillus foetidus SE4 |

La présente invention est illustrée par les exemples suivants.

### Exemple 1

### Isolement de la séquence codante de l'aspergillopepsine d'Aspergillus foetidus SE4

On réalise une culture de la souche d'Aspergillus foetidus ATCC 14916 dans un milieu nutritif liquide ACM ("Aspergillus Complete Medium") qui contient 2 % (poids/volume) d'extrait de malt, 0,1 % (poids/volume) de peptone (DIFCO) et 2 % (poids/volume) de glucose. Après une incubation de 72 heures à 32 °C, les spores sont récoltées.

Ces spores de la souche d'Aspergillus foetidus ATCC 14916 est soumis à un traitement de mutagénèse par ultra-violet selon la technique décrite dans PONTECORVO, G., ROPER, J.A., HEMMONS, L.M., MACDONALD, K.D., and BUFTON, A.W.J., Advances in Genetics 5 (1953), pages 141-238.

Les souches traitées sont étalées sur un milieu nutritif gélosé POTATO DEXTROSE AGAR (DIFCO) auquel a été ajouté l'amidon (5 % en poids/volume). Après une incubation de 48 heures à 32 °C, les colonies présentant le halo d'hydrolyse d'amidon le plus grand sont prélevées et repiquées sur le milieu nutritif gélose. On isole une souche produisant beaucoup de glucoamylase. Cette souche nommée SE4 présente des conidies brunes décolorées.

On réalise une culture de la souche d'Aspergillus foetidus SE4 dans le milieu nutritif liquide ACM. Après une incubation de 48 heures à 32 °C, le mycélium est récolté.

L'ADN génomique de la souche d'Aspergillus foetidus SE4 est isolé selon la technique décrite dans GARBER, R.C. and YODER, O.L., Anal. Biochem. 135 (1983), pages 416-422.

On réalise le mélange suivant :

| | |
|---|---|
| H₂O distillée | 62 µl |
| Tampon PCR (10x) | 10 µl |
| dNTPs (2,5 mM chacun) | 8 µl |
| Oligonucléotide SEQ ID NO:1 (20 µM) | 5 µl |
| Oligonucléotide SEQ ID NO:3 (20 µM) | 5 µl |
| ADN génomique (dilutions 10⁻¹ à 10⁻⁴) | 10 µl |
| Taq-polymérase (5 U/µl) | 0,2 µl |

Le tampon PCR, sous la citation "(10x) Reaction Buffer composition", l'abréviation dNTPs, qui signifie tous les nucléotides dATP, dTTP, dGTP et dCTP, et le produit Taq-polymérase sont décrits dans la notice du kit vendu sous le nom "GENEAMP DNA AMPLIFICATION REAGENT KIT with AMPLITAQ Recombinant Taq DNA Polymerase" (PERKIN ELMER CETUS).

L'ADN génomique isolé de la souche d'Aspergillus foetidus SE4, est mis en oeuvre dans ce mélange à une concentration de 1 µg/µl.

La séquence codante de l'aspergillopepsine d'Aspergillus foetidus SE4 a été isolée selon la technique de la "PCR" (technique de la "Polymerase Chain Reaction" décrite dans SAIKI, R.K., et al., SCIENCE, 239 (1988), pages 487-491).

Les séquences des oligonucléotides synthétiques mis en oeuvre lors de cette étude sont les suivantes : 
SEQ ID NO:1

- TGATGATCAGGATCCGGCGGCCGCACCTCAGCAATGGTCGTCTTCAGCAAAACCGCTGCCCTC - 3′

BamHI   NotI

SEQ ID NO:2

5′ - CTGGGATTCGCCGCTCAGGCTTAGATTATCAAGCTTCATTCTAGAGACGACGACGAC - 3′

HindIII   XbaI
SEQ ID NO:3
5' - GTCGTCGTCGTCTCTAGAATGAAGCTTGATAATCTAAGCCTGAGCGGCGAATCCCAG - 3'
La séquence SEQ ID NO:3 est le complément de la séquence SEQ ID NO:2.

La séquence de l'oligonucléotide SEQ ID NO:1 contient les informations pour les sites de restriction BamHI et NotI, les informations pour la partie finale de la région 5' non traduite de la glucoamylase d'Aspergillus foetidus SE4 et le début de la séquence codante de l'aspergillopepsine.

La séquence de l'oligonucléotide SEO ID NO:2 et, son complément, l'oligonucléotide SEQ ID NO:3 contiennent les nucléotides qui correspondent à la fin de la séquence codante de l'aspergillopepsine et les informations supplémentaires pour les sites de restriction HindIII et XbaI.

La technique utilisée pour construire les oligonucléotides synthétiques est décrite dans BEAUCAGE, S.L. et al (1981), Tetrahedron Letters, 22, pages 1859-1882 et en utilisant des β-cyanoéthyl phosphoramidites dans un appareil de BIOSEARCH CYCLONE SYNTHESIZER.

Les conditions suivies pour la "PCR" sont les suivantes : 2 minutes à 95 °C, puis 40 cycles d'une série comprenant 1 minute à 95 °C, 1 minute à 50 °C et 2 minutes à 72 °C, puis 10 minutes à 72 °C, puis une température de 20 °C est maintenue. Tous les autres paramètres utilisés pour cette "PCR" correspondent à ceux décrits dans la notice du kit vendu sous le nom "GENEAMP DNA AMPLIFICATION REAGENT KIT with AMPLITAQ Recombinant Taq DNA Polymerase" (PERKIN ELMER CETUS).

Un fragment d'ADN d'environ 1,35 kb (1 kb = 1 000 paires de bases) a été isolé comme décrit ci-dessus. Il a été comparé à l'aide de la technique de l'analyse par restriction [analyse décrite dans Molecular Cloning - a laboratory manual - MANIATIS et al., (Cold Spring Harbor Laboratory) 1982, pages 374-379] à la séquence de l'aspergillopepsine A produite par la souche d'Aspergillus awamori UVK143f. Cette séquence de l'aspergillopepsine A a été publiée par Berka R.M. et al., GENE, 86, 1990, pages 153-162 et corrigée par Berka R.M. et al., GENE, 96, 1990, page 313. Aucune différence essentielle n'a été trouvée.

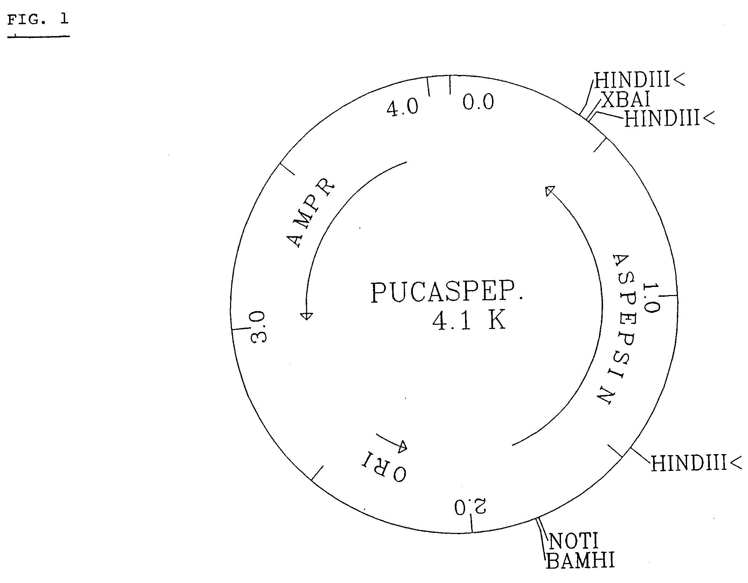

Le fragment d'ADN ainsi obtenu est digéré avec les enzymes de restriction BamHI et XbaI, puis est ligaturé, selon la technique de ligation décrite dans Molecular Cloning - a laboratory manual - SAMBROOK et al. - second edition, 1989, page 1.68-1.69, avec le plasmide pUC18 (CLONTECH laboratories, N° 6110-1), qui a été préalablement digéré aux sites BamHI et XbaI. Le vecteur pUCASPEP (figure 1) est ainsi obtenu.

### Exemple 2

### Construction du plasmide pFGA2MUT

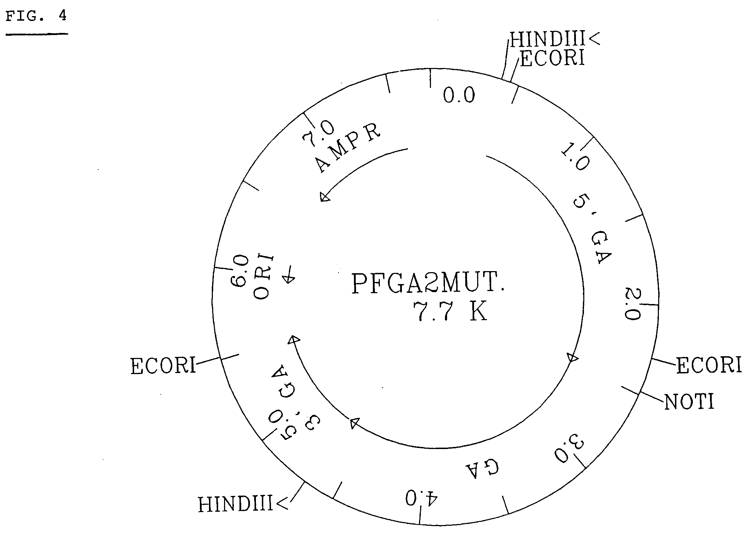

Le plasmide pFGA2MUT (figure 4) contient le gène de la glucoamylase d'Aspergillus foetidus SE4 dans lequel on a créé un site de clonage NotI entre le promoteur et la partie codante du gène de la glucoamylase, et un site de clonage HindIII entre la partie codante et le terminateur du gène de la glucoamylase. Ces deux sites de clonage NotI et HindIII sont créés par mutagénèse, comme décrit ci-après.

Le gène de la glucoamylase d'Aspergillus foetidus SE4 est isolé selon la description suivante.

L'ADN chromosomique de la souche d'Aspergillus foetidus SE4 est isolé selon la technique de GARBER et al., comme décrit à l'exemple 1. Cet ADN chromosomique isolé est digéré partiellement avec l'enzyme de restriction SauIIIA et les fragments ayant une taille de 8 à 10 kb sont isolés et purifiés par un gradient de sucrose (15 à 40 %) selon la technique décrite dans AUSUBEL, F.M. et al. (1989), Current Protocols in Molecular Biology (John WILLEY and Sons), pages 5.3.2-5.3.8. Ces fragments isolés et purifiés sont introduits dans le plasmide pBR322 (CLONTECH LABORATORIES catalogue n° 6210-1), qui a été préalablement digéré par l'enzyme de restriction BamHI. On transforme ensuite la souche d'E. coli DH5α [décrite par HANAHAN, D., J. Mol. Biol. (1983) 166, page 557, et vendue par BETHESDA RESEARCH LABORATORIES (BRL), catalogue B.R.L. Focus (1986) 8, page 9].

Environ 15 000 clones d'E. coli transformés sont criblés à l'aide de la technique d'hybridation (SAMBROOK et al., pages 9.52 - 9.55) en utilisant l'oligonucléotide synthétique suivant (SEQ ID NO:4) :
5' - GATTCATGGTTGAGCAACGAAGCGA - 3'
On se base sur la séquence de nucléotides publiée par BOEL et al., EMBO J. 3 (1984), pages 1097-1102.

On isole une colonie qui hybride avec cet oligonucléotide. On analyse les sites de restriction et on constate que cette souche contient le gène complet de la glucoamylase, c'est-à-dire le promoteur, la région codante ainsi que le signal de sécrétion, et le terminateur.

Le fragment de 8,7 kp a été introduit, comme décrit ci-dessus, dans le plasmide pBR322 au site BamHI.

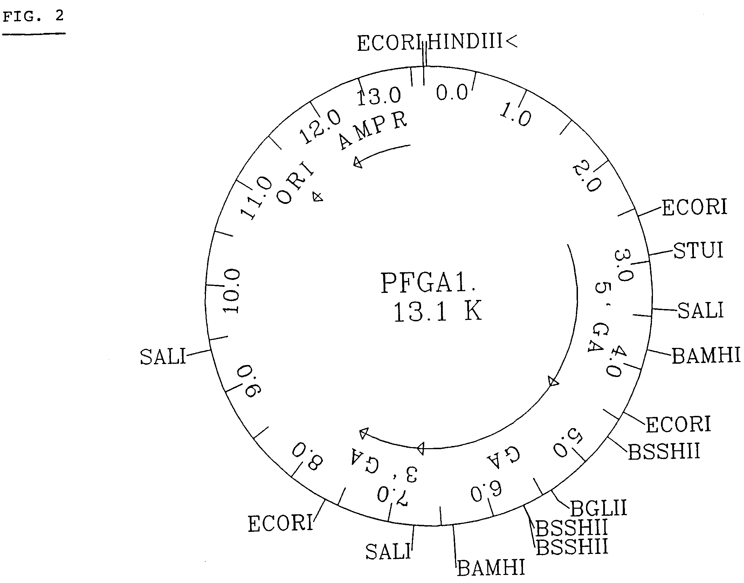

On crée ainsi le vecteur pFGA1 (figure 2), qui contient un insert d'ADN d'Aspergillus de 8,7 kb. Cet insert est la seule différence entre le plasmide pBR322 et le vecteur pFGA1.

On construit, comme suit, un plasmide qui est dérivé du vecteur pFGA1 et qui contient un insert moins grand que celui du vecteur pFGA1. Le vecteur pFGA1 est digéré par l'enzyme de restriction EcoRI, puis, à partir de ceci, on isole le fragment EcoRI d'une taille d'environ 5 kb contenant le promoteur, la région codante et le terminateur de la glucoamylase d'Aspergillus foetidus SE4 en suivant la méthode décrite par ZHU et al., 1985.

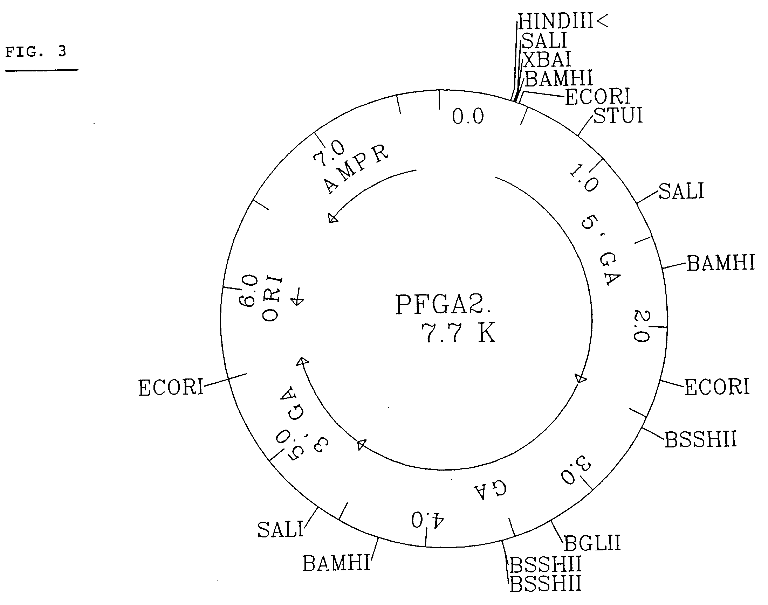

Ce fragment EcoRI est ensuite inséré, en suivant la technique décrite dans SAMBROOK et al., pages 1.68-1.69, dans le plasmide pUC18, qui a été préalablement digéré par l'enzyme de restriction EcoRI. On crée ainsi le vecteur pFGA2 (figure 3).

Les séquences nucléotidiques du promoteur et du terminateur de la glucoamylase d'Aspergillus foetidus SE4 ont été déterminées par la méthode de terminaison de chaînes utilisant des dideoxynucléotides, de SANGER et al. (1977) Proc. Natl. Acad. Sci. USA 74, p. 5463-5467.

L'analyse de cette séquence montre la présence d'un promoteur et d'un terminateur. On identifie la séquence de nucléotides (SEQ ID NO:7) codant pour le promoteur de la glucoamylase d'Aspergillus foetidus SE4. On identifie la séquence de nucléotides (SEQ ID NO:8) codant pour le terminateur de la glucoamylase d'Aspergillus foetidus SE4.

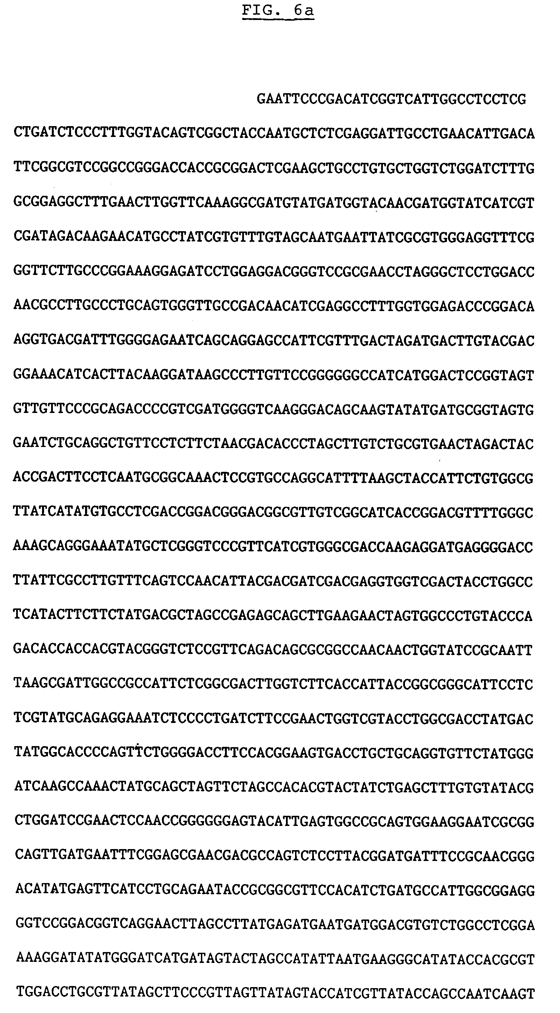

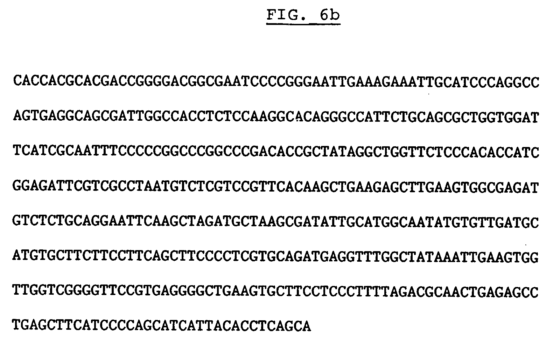

La figure 6 représente la séquence de nucléotides codant pour le promoteur de la glucoamylase d'Aspergillus foetidus SE4.

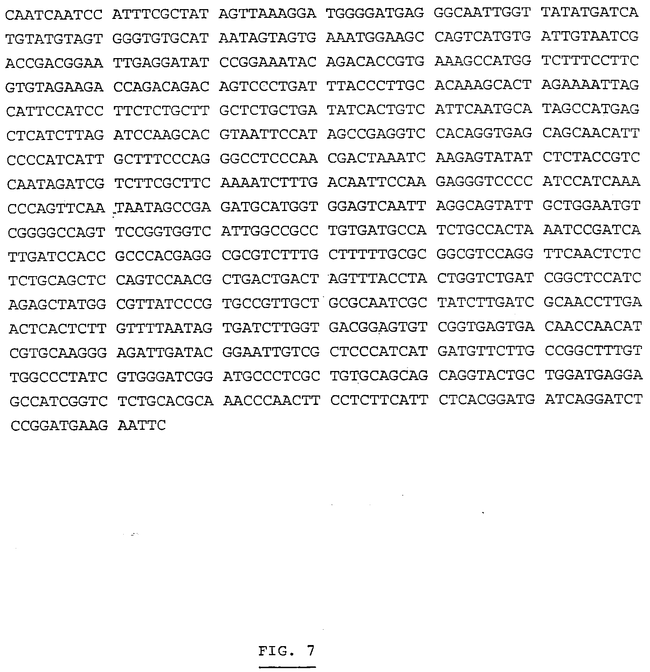

La figure 7 représente la séquence de nucléotides codant pour le terminateur de la glucoamylase d'Aspergillus foetidus SE4.

Les oligonucléotides synthétiques utilisés pour initier les réactions d'élongation par la T7 DNA polymérase ont été synthétisés par la méthode de BEAUCAGE et al. (1981) Tetrahedron letters 22:1859-1882. Le séquençage a été effectué suivant le protocole donné par le fournisseur du kit de séquençage (PHARMACIA), en procédant à une dénaturation de l'ADN double brin par traitement avec du NaOH.

La stratégie de séquençage est décrite par SAMBROOK, 1989, p. 13.15 et 13.17. Les gels de polyacrylamide pour le séquençage ont été effectués suivant la technique décrite par SAMBROOK, 1989, p. 13.45-13.58.

On construit, comme suit, un plasmide qui est dérivé du plasmide pFGA2 et qui contient le gène de la glucoamylase d'Aspergillus foetidus SE4 dans lequel le promoteur et le terminateur sont séparés de la partie codante par des sites de restriction NotI et HindIII. On crée ainsi deux sites de restriction NotI et HindIII dans le plasmide pFGA2, qui contient le promoteur et le terminateur. Le promoteur et le terminateur de la glucoamylase d'Aspergillus foetidus SE4 sont séparés par des sites de restriction NotI et HindIII créés par deux mutagénèses dirigées suivant la procédure décrite dans la notice technique du kit de mutagénèse (BIORAD) "MUTA-GENE PHAGEMID in vitro Mutagenesis Kit".

Les oligonucléotides synthétiques utilisés au cours de cette procédure sont les suivants, respectivement SEQ ID NO:5 et SEQ ID NO:6 : 
5′ - GCCTGAGCTTCATCCCCAGCGCGGCCGCATCATTACACCTCAGCAATGT - 3′
NotI
5′ - TGACTGACACCTGGCGGTGAAAGCTTCAATCAATCCATTTCGCTATAGTT - 3′
HindIII
On obtient ainsi le vecteur pFGA2MUT qui contient le site de restriction NotI au début de la région 5' non traduite du gène de la glucoamylase et le site de clivage HindIII après le stop-codon et dans la région 3' non traduite de la glucoamylase.

### Exemple 3

### Construction du vecteur d'intégration

Le plasmide pUCASPEP tel qu'obtenu à l'exemple 1 , est digéré partiellement avec l'enzyme de restriction HindIII, puis complétement avec l'enzyme de restriction NotI. Ainsi, un fragment de 1,3 kb contenant la séquence codante de l'aspergillopepsine et la région 5' non traduite du gène de la glucoamylase d'Aspergillus foetidus SE4, est isolé en suivant la technique décrite par ZHU, J.D. et al., BIO/TECHNOLOGY, 3, 1985, pages 1014-1016.

La digestion partielle et complète des plasmides avec des enzymes de restriction est effectuée en suivant la technique décrite par SAMBROOK et al. 1989, chapitres 5.28-5.32.

Ce fragment est introduit dans le site de clonage NotI-HindIII du plasmide pFGA2MUT, obtenu comme décrit à l'exemple 2. Ce plasmide contient le promoteur et le terminateur du gène de la glucoamylase d'Aspergillus foetidus SE4, séparés par les sites de clonage NotI et HindIII, créés par mutagénèse dirigée, comme décrit à l'exemple 2. La partie codante de la glucoamylase est donc remplacée par la partie codante de l'aspergillopepsine.

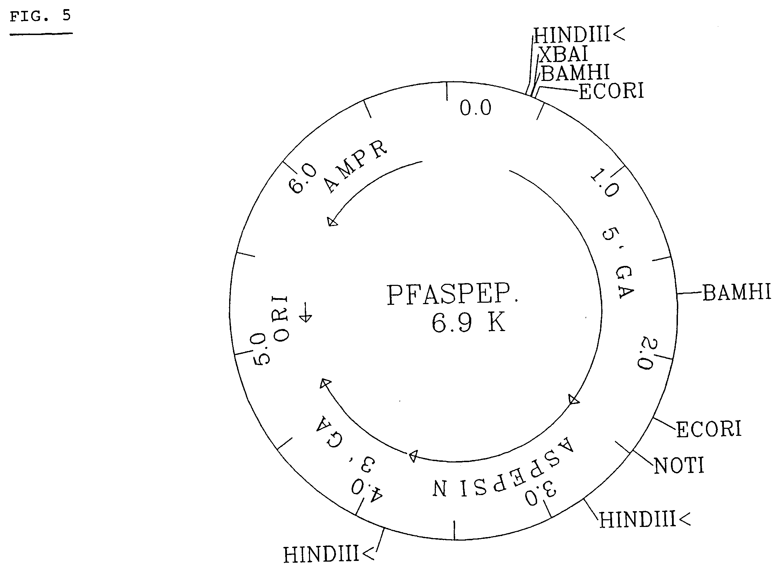

Le vecteur pFASPEP (figure 5) est ainsi obtenu. Ce vecteur pFASPEP contient la séquence codante de l'aspergillopepsine sous le contrôle des signaux de transcription de la glucoamylase d'Aspergillus foetidus. Ce vecteur contient le système d'expression qui comprend les séquences suivantes :
- la séquence du promoteur de la glucoamylase d'Aspergillus foetidus SE4,
- la séquence du signal de sécrétion de l'aspergillopepsine d'Aspergillus foetidus SE4,
- la séquence du propeptide de l'aspergillopepsine d'Aspergillus foetidus SE4,
- la séquence mature de l'aspergillopepsine d'Aspergillus foetidus SE4, et
- la séquence du terminateur de la glucoamylase d'Aspergillus foetidus SE4.

### Exemple 4

### Transformation de la souche d'Aspergillus foetidus SE4

Le vecteur pFASPEP, tel qu'obtenu à l'exemple 3, est ensuite introduit dans la souche d'Aspergillus foetidus SE4 (dont l'obtention est décrite à l'exemple 1) par cotransformation comme décrite dans CARREZ et al., GENE, 94 (1990), pages 147-154.

Le plasmide p3SR2, qui contient le gène de l'acétamidase d'Aspergillus nidulans (amdS), est utilisé comme marqueur sélectif.

Le plasmide p3SR2 est décrit dans Hynes, M.J., et al., Mol. Cell. Biol., 3 (1983), pages 1430-1439. On effectue la transformation avec ce plasmide sur des protoplastes selon la technique décrite par KELLY, J.M. and HYNES, M.J., EMBO J., 4 (1985), pages 475-479.

Lors de la transformation, on utilise 10 fois plus de vecteur pFASPEP que de plasmide p3SR2.

Environ 100 souches transformées sont analysées. Ces souches transformées sont sélectionnées sur le milieu gélosé Y contenant du lait écrémé pour détecter la production d'aspergillopepsine. Les colonies des souches transformées qui sécrètent l'aspergillopepsine dans le milieu de culture, sont entourées d'un halo après 1 jour d'incubation à 34 °C.

Le milieu gélosé Y est formé du milieu CZAPEK DOX AGAR (DIFCO) auquel on ajoute 2 % (poids/poids) de lait écrémé (BACTO SKIM MILK de DIFCO), 0,3 % (poids/poids) de produit PHYTAGEL (SIGMA) dans un tampon citrate-phosphate 0,05 M à pH 3,0.

Environ 30 % des souches transformées sécrètent l'aspergillopepsine, elles ont donc intégré, dans leur génome, le vecteur de sélection p3SR2 et le vecteur d'intégration pFASPEP.

### Exemple 5

### Isolement et purification des souches transformées

Les souches transformées ainsi obtenues sont isolées et repiquées sur un milieu gélosé POTATO DEXTROSE AGAR (DIFCO). Elles sont mises en culture à 32 °C jusqu'à sporulation.

Les souches transformées produisant le plus d'aspergillopepsine sont isolées et repiquées sur un milieu gélosé POTATO DEXROSE AGAR (DIFCO). 40 souches transformées sont mises en culture à 32 °C sur ce milieu jusqu'à sporulation.

Les protoplastes transformés comme décrits à l'exemple 4, peuvent contenir plusieurs noyaux par cellule. Les souches transformées peuvent donc être hétérozygotes. Il est donc nécessaire de purifier ces souches transformées à partir de spores isolées contenant un seul noyau par cellule.

Les spores sont récoltées, diluées dans de l'eau physiologique puis étalées sur le milieu gélosé A contenant du lait écrémé, tel que décrit à l'exemple 4, de telle sorte que des colonies individuelles soient obtenues. Les colonies venant d'une seule spore et présentant un halo large sont repiquées de nouveau sur le milieu gélosé POTATO DEXTROSE AGAR. Ces colonies sont mises en culture à 32 °C sur ce milieu gélosé jusqu'à sporulation.

Les conidies purifiées des souches transformées sont récoltées et conservées. Ces souches transformées d'Aspergillus foetidus, ainsi obtenues, sont dénommées SE4tra.

### Exemple 6

### Production d'aspergillopepsine par la souche transformée d'Aspergillus foetidus SE4tra

Les conidies purifiées, obtenues à l'exemple 5, sont mises en culture dans un milieu liquide riche contenant de l'extrait de malt (DIFCO) (2 % en poids), de la peptone (DIFCO) (0,1 % en poids), de l'amidon hydrolysé (10 % en poids) et du carbonate de calcium (2 % en poids). Le pH du milieu de culture est ajusté à un pH 6 par l'addition d'ammoniac. La culture est réalisée à 32 °C sous agitation et est suivie durant 7 jours.

Puis, la culture obtenue est centrifugée à 5 000 tours/minute pendant 15 minutes (BECKMAN JA-10). On mesure l'activité enzymatique (aspergillopepsine) du surnageant après 4, 5, 6 et 7 jours de culture.

On mesure l'activité enzymatique selon une technique modifiée par rapport à la technique décrite par ICHISHIMA (Methods in Enzymology, vol. XIX, Proteolytic Enzymes, eds G. PERLMANN and L. LORAND, 1970, pages 397-399). La solution contenant le substrat est une solution aqueuse comprenant 2 % (poids/volume) de caséine, le pH de cette solution est ajusté à un pH de 2,7 par l'addition d'HCl concentré. Les réactifs suivants sont mis en oeuvre : 0,4 M de TCA (acide trichloroacétique), 0,4 M de Na₂CO₃ et le réactif de FOLIN-CIOCALTEAU (MERCK). Les réactifs commerciaux sont dilués 1:5 fois avec de l'eau distillée avant leur utilisation. Une solution standard de tyrosine est également préparée. L'échantillon de surnageant est dilué dans un tampon acétate-HCl 0,1 M (pH 2,7). Cet échantillon dilué est mélangé avec la solution de substrat comprenant la caséine dans une proportion de 1:1. Ce mélange est incubé à 30 °C durant 10 minutes. La réaction est arrêtée par l'addition d'un volume de TCA 0,4 M. Cette solution est centrifugée 10 minutes à 12 500 tours par minute (centrifugeuse BECKMAN JA-10). A 0,2 ml du surnageant de centrifugation, on ajoute 1 ml de Na₂CO₃ 0,4 M et 0,2 ml de réactif de FOLIN-CIOCALTEAU. Cette solution est incubée à 30 °C durant 25 minutes. Ensuite, on mesure l'absorbance à 660 nm.

L'activité enzymatique est exprimée en µmoles d'équivalents de tyrosine produits par minute durant l'hydrolyse de la caséine à un pH de 2,7 à 30 °C, déterminées par l'augmentation de l'absorbance mesurée à 660 nm.

Les résultats d'activité enzymatique obtenus sont comparés avec ceux de la souche d'Aspergillus foetidus non transformée (souche SE4). La production d'aspergillopepsifle de la souche transformée (souche SE4tra) en comparaison avec celle de la souche non transformée (souche SE4) est multipliée par un facteur d'environ 40.

On observe donc une forte augmentation de la production d'aspergillopepsine.

La production d'aspergillopepsine est substantiellement extracellulaire.

### Exemple 7

### Estimation du poids moléculaire de l'aspergillopepsine produite par la souche transformée SE4tra

L'aspergillopepsine produite par la souche transformée SE4tra est analysée par la technique SDS-PAGE, selon la méthode décrite dans Anal. Biochem. 78, 1977, pages 459-482 et Biochemistry 12, 1973, pages 871-890, en utilisant le système PHAST SYSTEM (LKB-PHARMACIA).

Pour ce faire, on effectue sur le surnageant tel qu'obtenu à l'exemple 6, une précipitation au moyen d'acide trichloroacétique (10 % v/v final). Le précipité obtenu est repris dans un tampon composé de 10 mM TRIS/HCl (pH = 8,0) (tris(hydroxyméthyl)aminométhane acidifié avec HCl), 1 mM EDTA (acide éthylènediamine tétraacétique), 2,5 % (poids/volume) SDS (sodium dodécyle sulfate), 5 % (volume/volume) β-mercaptoéthanol et 0,01 % (poids/volume) de bleu de bromophénol.

4 µl du précipité repris dans le tampon sont déposés sur un gel de polyacrylamide. Le système de gel utilisé est le système PHAST SYSTEM avec des gels contenant un gradient de polyacrylamide de 10-15 % (v/v) en présence de SDS. Les conditions d'électrophorèse sont celles prescrites par le fournisseur. Une coloration du gel au bleu de Coomassie fait apparaître un polypeptide d'un poids moléculaire d'environ 43 KDaltons.

Par cette analyse, on remarque que la taille de l'aspergillopepsine produite par la souche transformée correspond à la taille de l'aspergillopepsine native produite par la souche non transformée.

### Exemple 8

### Profil d'activité en fonction du pH pour l'aspergillopepsine produite par la souche transformée SE4tra

On mesure l'activité enzymatique de l'aspergillopepsine à différents pH (de 1,5 à 3,5) à une température de 30 °C en tampon acétate-HCl 0,1 M en présence de caséine. L'incubation dure 10 minutes. Les conditions appliquées sont celles décrites à l'exemple 6.

On met en oeuvre le surnageant obtenu à l'exemple 6. Ce surnageant est dilué de 50 à 500 fois dans le tampon acétate-HCl 0,1 M en fonction du pH et de l'activité enzymatique du surnageant.

Les résultats sont rassemblés dans le tableau 2.

Au cours de cet essai l'activité enzymatique maximale a été mesurée pour l'échantillon placé à un pH d'environ 3,0 et à une température de 30 °C. Par définition on a donc attribué à cet échantillon une activité relative de 100 %.

Cet exemple montre que l'aspergillopepsine présente une activité enzymatique optimale mesurée à une température d'environ 30 °C dans une gamme de pH comprise entre 2,5 et 3,5.

**TABLEAU 2**

| pH | Activité relative en % |
|---|---|
| 1,5 | 1 |
| 2,0 | 39 |
| 2,5 | 80 |
| 2,7 | 93 |
| 3,0 | 100 |
| 3,5 | 85 |

Le profil d'activité en fonction du pH de l'aspergillopepsine produite par la souche transformée est similaire à celui de l'aspergillopepsine produite par la souche non transformée, c'est-à-dire l'enzyme native.

### Exemple 9

### Profil d'activité en fonction de la température pour l'aspergillopepsine produite par la souche transformée SE4tra

On mesure l'activité enzymatique de l'aspergillopepsine à différentes températures (de 40 à 65 °C) à un pH de 2,7 en tampon acétate-HCl 0,1 M en présence de caséine. L'incubation dure 10 minutes. Les conditions appliquées sont celles décrites à l'exemple 6.

On met en oeuvre le surnageant tel qu'obtenu à l'exemple 6. Ce surnageant est dilué de 50 à 1 000 fois dans le tampon acétate-HCl 0,1 M en fonction du pH et de l'activité enzymatique du surnageant.

Les résultats sont rassemblés dans le tableau 3.

Au cours de cet essai l'activité enzymatique maximale a été mesurée pour l'échantillon placé à un pH d'environ 2,7 et à une température de 55 °C. Par définition on a donc attribué à cet échantillon une activité relative de 100 %.

Cet exemple montre que l'aspergillopepsine présente une activité enzymatique optimale mesurée à un pH d'environ 2,7 dans une gamme de température comprise entre 50 et 60 °C.

**TABLEAU 3**

| température °C | Activité relative % |
|---|---|
| 40 | 46 |
| 45 | 66 |
| 50 | 90 |
| 55 | 100 |
| 60 | 88 |
| 65 | 39 |

Le profil d'activité en fonction de la température de l'aspergillopepsine produite par la souche transformée est similaire à celui de l'aspergillopepsine produite par la souche non transformée, c'est-à-dire l'enzyme native.

Les exemples 7, 8 et 9 montrent que I'aspergillopepsine obtenue selon l'invention est glycosylée de la même manière que l'enzyme native. En effet, les caractéristiques de l'aspergillopepsine produite par la souche transformée sont similaires à celles de l'aspergillopepsine produite par la souche non transformée.

## Revendications

1. Système d'expression utilisable pour la production extracellulaire d'une protéase aspartique microbienne, caractérisé en ce qu'il comprend :
- la séquence d'un promoteur,
- la séquence d'un signal de sécrétion,
- la séquence d'un propeptide,
- la séquence mature de la protéase aspartique microbienne, et
- la séquence d'un terminateur.

2. Système d'expression selon la revendication 1, caractérisé en ce que la séquence du signal de sécrétion et la séquence du propeptide sont celles de la protéase aspartique microbienne.

3. Système d'expression selon la revendication 1 ou 2, caractérisé en ce que le promoteur est celui de la glucoamylase et le terminateur est celui de la glucoamylase.

4. Système d'expression selon la revendication 1, 2 ou 3, caractérisé en ce que la protéase aspartique microbienne est une protéase aspartique fongique.

5. Système d'expression selon la revendication 4, caractérisé en ce que la protéase aspartique microbienne est une aspergillopepsine.

6. Système d'expression selon l'une quelconque des revendications précédentes, caractérisé en ce que la séquence du promoteur provient de la glucoamylase d'une souche de champignon filamenteux.

7. Système d'expression selon l'une quelconque des revendications précédentes, caractérisé en ce que la séquence mature de la protéase aspartique microbienne provient d'une souche de champignon filamenteux.

8. Système d'expression selon la revendication 6 ou 7, caractérisé en ce que la souche de champignon filamenteux est une souche d'Aspergillus.

9. Système d'expression selon la revendication 8, caractérisé en ce que la souche d'Aspergillus est une souche d'Aspergillus foetidus.

10. Système d'expression selon la revendication 3, caractérisé en ce qu'il comprend :
- la séquence du promoteur de la glucoamylase d'Aspergillus foetidus SE4,
- la séquence du signal de sécrétion de la protéase aspartique microbienne d'Aspergillus foetidus SE4,
- la séquence du propeptide de la protéase aspartique microbienne d'Aspergillus foetidus SE4,
- la séquence mature de la protéase aspartique microbienne d'Aspergillus foetidus SE4, et
- la séquence du terminateur de la glucoamylase d'Aspergillus foetidus SE4.

11. Vecteur d'intégration contenant le système d'expression selon l'une quelconque des revendications précédentes, et apte à permettre l'expression de la protéase aspartique microbienne.

12. Vecteur d'intégration selon la revendication 11, caractérisé en ce qu'il s'agit du plasmide pFASPEP.

13. Cellule de champignon filamenteux transformée par le vecteur d'intégration selon la revendication 11 ou 12.

14. Cellule transformée selon la revendication 13, caractérisée en ce que la cellule de champignon filamenteux est une cellule d'Aspergillus.

15. Cellule transformée selon la revendication 14, caractérisée en ce que la cellule d'Aspergillus est une cellule d'Aspergillus foetidus.

16. Cellule transformée selon la revendication 15, caractérisée en ce que la cellule d'Aspergillus est une cellule d'Aspergillus foetidus SE4.

17. Souche isolée et purifiée d'Aspergillus foetidus SE4tra.

18. Promoteur de la glucoamylase d'Aspergillus foetidus.

19. Molécule d'ADN comprenant la séquence de nucléotides SEQ ID NO:7 qui code pour le promoteur de la glucoamylase d'Aspergillus foetidus SE4.

20. Terminateur de la glucoamylase d'Aspergillus foetidus.

21. Molécule d'ADN comprenant la séquence de nucléotides SEQ ID NO:8 qui code pour le terminateur de la glucoamylase d'Aspergillus foetidus SE4.

22. Procédé pour la production extracellulaire de protéase aspartique microbienne, caractérisé en ce qu'il comprend les étapes suivantes :
- transformation d'une cellule par un vecteur d'intégration contenant un système d'expression selon l'une quelconque des revendications 1 à 10 dans des conditions permettant l'expression et la sécrétion de la protéase aspartique microbienne,
- culture de la cellule transformée dans un milieu de culture adéquat, et
- récupération de la protéase aspartique microbienne sécrétée.

23. Protéase aspartique microbienne produite par une cellule transformée selon l'une quelconque des revendications 13, 14, 15, 16 ou 17.

24. Protéase aspartique microbienne dérivée d'une souche d'Aspergillus foetidus transformée par le vecteur d'intégration selon la revendication 11 ou 12.

25. Protéase aspartique microbienne dérivée de la souche d'Aspergillus foetidus SE4 transformée par le vecteur d'intégration selon la revendication 11 ou 12.

26. Protéase aspartique microbienne selon la revendication 24, caractérisée en ce qu'il s'agit de l'aspergillopepsine.
